# EUROPEAN PATENT SPECIFICATION

(11) **EP 2 408 471 B1**
(45) Date of publication and mention of the grant of the patent: **24.06.2015**
(21) Application number: 10753003.2
(22) Date of filing: 19.03.2010
(51) Int. Cl.: A61K 38/30, A61K 38/18, A61P 17/02, A61K 38/38, A61K 38/39, C12Q 1/52, G01N 33/74, A61K 38/45

(54) **TARGETS FOR GROWTH FACTOR SIGNALLING AND METHODS OF THERAPY**
ZIELMOLEKÜLE FÜR WACHSTUNGSFAKTORSIGNALWEGE UND THERAPIEVERFAHREN
CIBLES POUR LA SIGNALISATION DES FACTEURS DE CROISSANCE ET PROCÉDÉS THÉRAPEUTIQUES

(30) Priority: 19.03.2009 AU 2009901181
(43) Date of publication of application: 25.01.2012
(73) Proprietor: Queensland University Of Technology, Brisbane, QLD 4000 (AU)
(72) Inventor: CROLL, Tristan, Bald Hills, QLD 4036 (AU); SHOOTER, Gary Keith, East Ipswich, QLD 4305 (AU); RIZZI, Simone, 6883 Novazzano (CH); UPTON, Zee, Kenmore Hills, QLD 4069 (AU); PEET, Jesse, Deception Bay, QLD 4508 (AU); VAN LONKHUYZEN, Derek, Morayfield, QLD 4506 (AU)
(74) Representative: Sutcliffe, Nicholas Robert
(86) International application number: PCT/AU2010/000316
(87) International publication number: WO 2010/105302

(56) References cited:
- WO-A1-98/04245
- WO-A1-2004/069871
- WO-A1-2007/069817
- WO-A1-2007/093008
- WO-A1-2008/144933
- WO-A2-02/11747
- WO-A2-2006/100679
- WO-A2-2008/098129
- US-A1- 2005 208 612
- US-A1- 2008 020 982
- K. SAKAI: "Tissue Transglutaminase Facilitates the Polymerization of Insulin-like Growth Factor-binding Protein-1 (IGFBP-1) and Leads to Loss of IGFBP-1's Ability to Inhibit Insulin-like Growth Factor-I-stimulated Protein Synthesis", JOURNAL OF BIOLOGICAL CHEMISTRY, vol. 276, no. 12, 19 December 2000 (2000-12-19), pages 8740-8745, XP055066225, ISSN: 0021-9258, DOI: 10.1074/jbc.M008359200
- B. G. HOLLIER ET AL: "Substrate-Bound Insulin-Like Growth Factor (IGF)-I-IGF Binding Protein-Vitronectin-Stimulated Breast Cell Migration Is Enhanced by Coactivation of the Phosphatidylinositide 3-Kinase/AKT Pathway by v-Integrins and the IGF-I Receptor", ENDOCRINOLOGY, vol. 149, no. 3, 6 December 2007 (2007-12-06), pages 1075-1090, XP055065915, ISSN: 0013-7227, DOI: 10.1210/en.2007-0740
- SIEGEL ET AL: "Transglutaminase 2 inhibitors and their therapeutic role in disease states", PHARMACOLOGY AND THERAPEUTICS, ELSEVIER, GB, vol. 115, no. 2, 13 July 2007 (2007-07-13) , pages 232-245, XP022152216, ISSN: 0163-7258, DOI: 10.1016/J.PHARMTHERA.2007.05.003
- MANASWINI SIVARAMAKRISHNAN ET AL: "Transglutaminases and receptor tyrosine kinases", AMINO ACIDS ; THE FORUM FOR AMINO ACID AND PROTEIN RESEARCH, SPRINGER-VERLAG, VI, vol. 44, no. 1, 11 October 2011 (2011-10-11), pages 19-24, XP035156546, ISSN: 1438-2199, DOI: 10.1007/S00726-011-1113-X

## Description

### FIELD OF THE INVENTION

THIS INVENTION relates to therapy and/or prevention of transglutaminase-related diseases. More particularly, this invention relates to targeting an interaction between a transglutaminase and an insulin-like growth factor and/or an insulin-like growth factor receptor family member for development of therapeutic agents and methods of treatment using the same.

### BACKGROUND TO TIRE INVENTION

The insulin-like growth factor (IGF) axis is a complex network, consisting of various isoforms of the two ligands IGF-I and IGF-II, a range of cell membrane receptors, and six high-affinity soluble binding proteins which in turn bind to other active ligands.³ This network is essential for normal growth and development, and in particular the proper development and functioning of the immune, lymphatic³ and musculoskeletal⁴⁻⁶ systems, and in wound healing.⁷ On the other hand, breakdowns in regulation of IGF pathways are implicated in cancers,⁸ atherosclerosis⁹ and impaired wound healing,¹⁰ amongst other pathological states.

For these reasons, the IGF network is the subject of a great deal of research, particularly with respect to the potential for treatments for conditions such as heart disease,⁹ chronic wounds,^{7, 10} amyotrophic lateral sclerosis,⁶ muscular dystrophy¹¹ and cancer.⁸ Moreover, complexes of IGF-I, vitronectin (VN) and IGF binding proteins (IGFBPs) have been used as potent stimulators of reepithelialisation and they act via co-activation of the IGF-I receptor (IGF1R) and the vitronectin-binding αᵥ integrins**.**¹²

### SUMMARY OF THE INVENTION

In a first aspect, the invention provides a method of screening, for a therapeutic agent effective for the treatment of a transglutaminase-associated disorder, disease and/or condition, said method including the step of determining whether a candidate agent can modulate an interaction between
(i) a transglutaminase (TG) and an insulin-like growth factor (IGF);
   or
(ii) a TO and a member of IGF receptor family ,said method being set out in claim 1.

A therapeutic agent effective for treatment of a transglutaminase-associated disorder, disease and/or condition, may be designed, engineered, screened or otherwise produced according to a method of the first aspect.

A pharmaceutical composition for treating a transglutaminase-associated disorder, disease and/or condition, may comprise a therapeutic agent as described above, together with a pharmaceutically-acceptable diluent, carrier or excipient.

A pharmaceutical composition for treating a transglutaminase-associated disorder, disease and/or condition may comprise a therapeutic agent selected from the group consisting of:
(i) an isolated TG, or a fragment thereof;
(ii) an isolated TG substrate, or a fragment thereof;
(iii) an isolated IGF amino acid sequence, or an analogue thereof, together with an isolated polypeptide, or a fragment thereof, that binds to or interacts with an extracellular matrix; and
(iv) a modulator of an interaction between a TG and an IGF, and a pharmaceutically-acceptable diluent, carrier or excipient.

The isolated TG substrate may be selected from an acyl-donor substrate and an acyl-acceptor substrate.

The acyl-donor substrate may comprise a glutamine residue.

The acyl acceptor substrate may comprise a lysine residue

A method of treating a transglutaminase-associated disorder, disease and/or condition in an animal may include the step of administering to said animal:
a therapeutic agent effective for treatment of a transglutaminase-associated disorder, disease and/or condition as described above;
   and/or
a pharmaceutical composition as described above
to thereby treat the transglutaminase-associated disorder, disease and/or condition.
A method of treating a transglutaminase-associated disorder, disease and/or condition, may include
   the step of modulating an interaction between
   (i) a TG and an IGF; and/or
   (ii) a TG and a member of IGF receptor family,
to thereby treat a transglutaminase-associated disorder, disease and/or condition.

A modulator may modulate an interaction between a TG and an IGF and/or a TG and a member of IGF receptor family to thereby modulate cell migration and/or proliferation.

The method of modulating cell migration and/or proliferation may be a method of promoting cell migration and/or proliferation.

The method of promoting cell migration and/or proliferation may relate to promotion or induction of epithelial cell migration and/or proliferation to facilitate wound healing, treatment of ulcers, burns and the like and/or skin regeneration in mammals, preferably humans.

The method of modulating cell migration and/or proliferation may be a method of preventing cell migration and/or proliferation.

The method of preventing cell migration and/or proliferation may relate to prevention or inhibition in cancer cell metastasis, hyperproliferative disorders such as scarring, psoriasis and atherosclerosis.

The method of modulating cell migration and/or proliferation may relate to modulating cell migration and/or proliferation in tumour resistance to chemotherapy.

The method may relate to an animal.

The animal may be a mammal.

The mammal may be a human

The methods described above may relate to prophylactic and/or therapeutic methods of treatment.

An isolated protein complex may comprise :
(i) a TG, or fragment thereof and an IGF, or fragment thereof;
(ii) a TG, or fragment thereof and a member of IGF receptor family, or fragment thereof.

The IGF may be selected from IGF-I and IGF-II

The IGF may be IGF-I.

IGF receptor family member may be selected from the group consisting of IGF-1 receptor, insulin receptor, insulin receptor related receptor and insulin-IGF1 hybrid receptor.

The IGF receptor family member lay be selected from IGF-1 receptor, insulin receptor and insulin-IGF1 hybrid receptor.

The IGF receptor family member may be GF-1 receptor. The TG may be
selected from the group consisting of FXIII, TG1, TG2, TG3, TG4, TG5, TG6 and TG7.

The TG may be selected from FXIII and TG2.

The TG may be TG2.

The modulator described above may be selected from the group consisting of an isolated peptide, an isolated polypeptide, an isolated nucleic acid and a small organic molecule. The isolated nucleic acid may encode a modulator which is a protein or alternatively, the isolated nucleic acid may itself have modulator activity such as, but not limited to, an RNAi molecule or a ribozyme.

The modulator may be an antibody, and more preferably a monoclonal antibody. The antibody may bind to and/or have been raised against a TG.

The antibody may be bind to and/or have been raised against an IGF or a member of IGF receptor family.

In methods that relate to a member of the IGF receptor family, the modulator may be an antibody

The modulator may be an isolated protein complex comprising an IGF, or at least a domain of an IGF which is capable of binding a cognate IGF receptor and vitronectin (VN) or fibronectin (FN), or at least an integrin-binding domain of VN or FN.

In preferred embodiments the modulator is a selective modulator.

In preferred embodiments the modulator is selected from an activator and an inhibitor.

In preferred embodiments, the activator is an activator of a GDP-inhibited TG.

In other preferred embodiments, the activator is selected from an isolated peptide, an isolated polypeptide and an isolated protein complex, comprising a polyanionic amino acid sequence, wherein
the polyanionic amino acid sequence is a polyanionic domain of VN, and
the polyanionic domain of VN is the polyanionic region corresponding to amino acids 53-64 of mature VN (SEQ ID NO:2).

In preferred embodiments the modulator modulates a substrate site for a TG. More preferably, the substrate site comprises an amino acid residue selected from a lysine residue and glutamine residue.

In certain preferred embodiments, the modulator is an inhibitor of a substrate donor site for a TG.

Preferably, the modulator is a competitive inhibitor for a substrate donor site such as a monofunctional primary amine.

Yet even more preferably, the monofunctional primary amine is monodansylcadaverine.

In other preferred embodiments, the inhibitor is a non-crosslinkable isolated IGF and/or member of IGF receptor family.

Preferably, the non-crosslinkable isolated IGF is selected from IGF-I and IGF-II.

In preferred embodiments, the non-crosslinkable isolated IGF is an isolated mutant IGF-I comprising a mutation of a lysine residue with respect to a wild-type IGF-I amino acid sequence. Preferably, the lysine residue is selected from the group consisting of lysine 27, lysine 65 and lysine 68.

More preferably, the lysine residue is lysine 68.

In other preferred embodiments, the non-crosslinkable isolated IGF-I is an isolated mutant IGF-I comprising a mutation of a glutamine residue with respect to a wild-type IGF-I amino acid sequence. Preferably, the glutamine residue is selected from the group consisting of glutamine 15 and glutamine 40 with respect to a wild-type IGF amino acid sequence.

In other preferred embodiments, the modulator is a non-crosslinkable IGF receptor family member. In preferred embodiments that relate to IGF1R, the isolated mutant IGF1R is an isolated mutant comprising a mutation of a glutamine donor residue. More preferably, the glutamine donor site in an IGF1R is selected from the group consisting glutamine 14, glutamine 15, glutamine 399, glutamine 400, glutamine 287, glutamine 318, glutamine 321, glutamine 396, glutamine 511, glutamine 596, glutamine 619 and glutamine 623 with respect to a wild-type IGF1R amino acid sequence.

In other preferred embodiments that realted IGF1R, the isolated mutant IGF1R is an isolated mutant comprising a mutation of a lysine residue. Preferably, the lysine residue is selected from the group consisting of lysine 159, lysine 191, lysine 498, lysine 530 and lysine 600, with respect to a wild-type IGF1R amino acid sequence.

In preferred embodiments of any one of the above, the mutation is selected from an insertion, a substitution and a deletion. More preferably the mutation is a substitution.

In further preferred embodiments, the inhibitor is a TG specific inhibitor. In particularly preferred embodiments, the TG specific inhibitor comprises a 3-halo-4,5-dihydroisoxazole moiety.

A pharmaceutical composition may further comprise an IGF. More preferably, the IGF is selected from IGF-I and IGF-II. Even more preferably, the IGF is IGF-I.

The pharmaceutical composition may be capable of treating or preventing a transglutaminase-associated disorder, disease and/or condition.

In preferred embodiments the transglutaminase-associated disorder, disease and/or condition is a cell migration and/or proliferation-associated disorder, disease and/or condition.

In preferred embodiments, the cell migration and/or proliferation-associated disorder, disease and/or condition is selected from the group consisting of wound healing, psoriasis, atherosclerosis, cancer, tumour resistance to chemotherapy, a burn, an ulcer and hypertrophic scarring.

In other preferred embodiments, the transglutaminase-associated disorder, disease and/or condition is an autoimmune disease.

More preferably, the autoimmune disease is diabetes and even more preferably, type I diabetes.

Although the invention is preferably directed to humans, it will be appreciated that the invention is also applicable to other mammals such as livestock, performance animals, domestic pets and the like.

Throughout this specification, unless the context requires otherwise, the words "comprise", "comprises" and "comprising" will be understood to imply the inclusion of a stated integer or group of integers but not the exclusion of any other integer or group of integers.

### BRIEF DESCRIPTION OF FIGURES

In order that the invention may be readily understood and put into practical effect, preferred embodiments will now be described by way of example with reference to the accompanying:

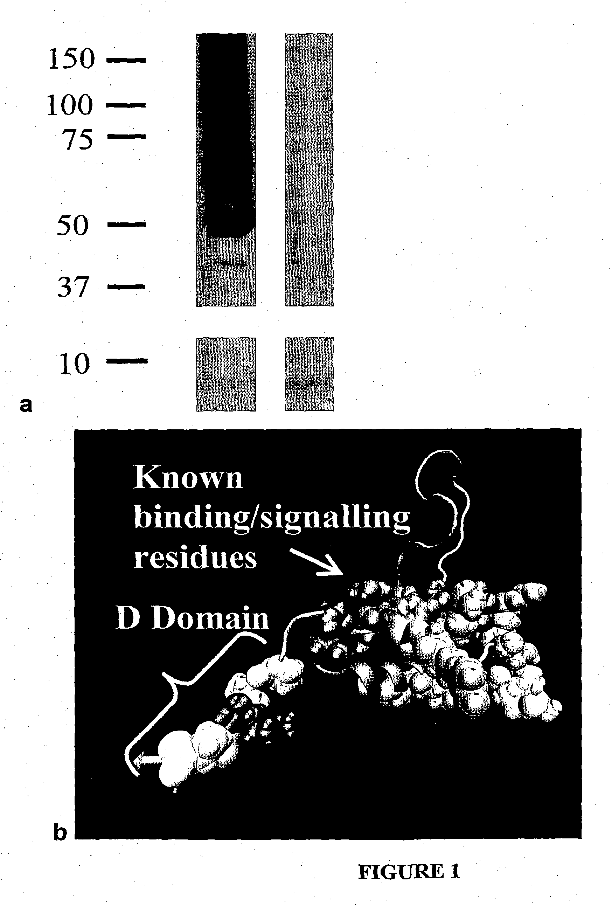

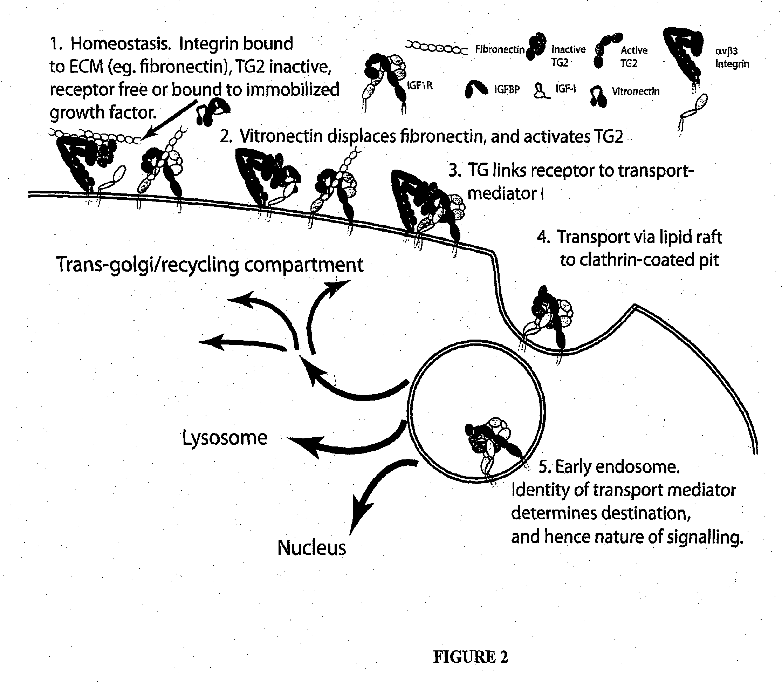

FIGURE 1 (a) IGF-I Western blot after reaction for 60 minutes with FXIII and PEG derivatized with a glutamine (left lane) or lysine FXIII substrate (right lane). This result indicates that IGF-I contains a lysine-donor substrate for transglutaminases. (b) 3D structure of IGF-I, with the putative FXIII site at the D domain shown relative to residues identified as important for receptor binding. FIGURE 2 Overview of the hypothesized mechanism of TG2-mediated IGF-I signalling.

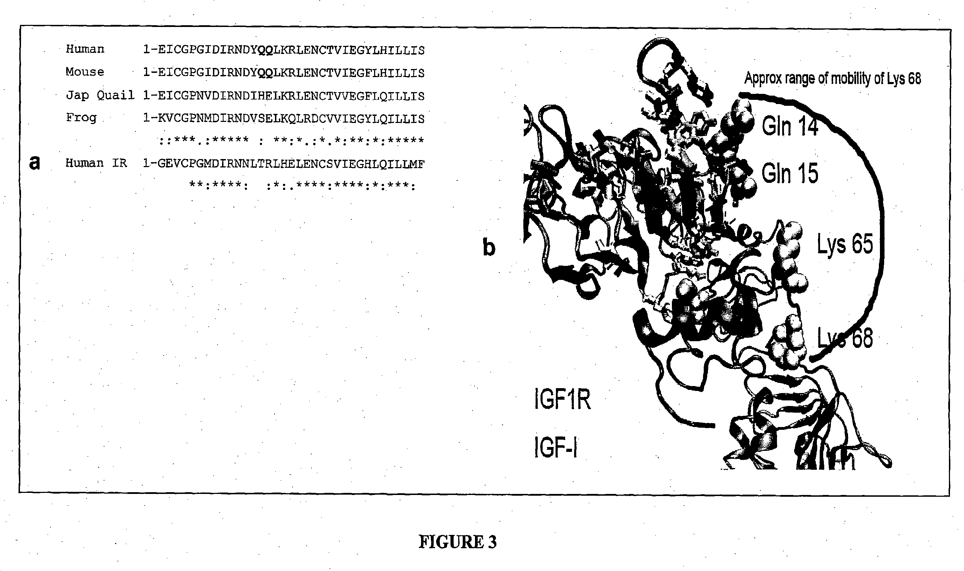

FIGURE 3 (a) BLAST/CLUSTAL W alignment of the N-terminal sequence of human IGF1R (top; SEQ ID NO:4 ) with the mouse (SEQ ID NO:5 ), quail (SEQ ID NO.6 ), and frog (SEQ ID NO:7 ), and with the human IR (bottom; SEQ ID NO:8 ). Human IR is compared directly to the human IGF1R. Glutamine residues at positions 14 and 15 appear to be a relatively recent addition, occurring after the divergence of mammals and birds. (b) Representation of the model of the IGF1R-IGF-I complex,¹ with the IGF-I D-domain Lys residues and the IGF1R Gln 14 and 15 shown as space-filling. All other residues within the N-terminal domain of the IGF1R that differ from their counterparts in the human IR are indicated as sticks. A significant amount of variation occurs in a surface patch surrounding Gln 14 and 15.

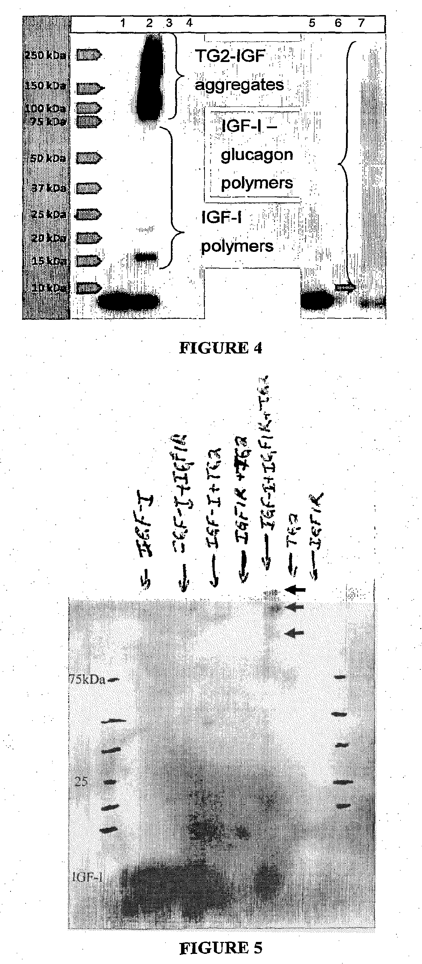

FIGURE 4 Reaction of IGF-I with glucagon catalysed by TG2. Lane 1: IGF-I control; 2: IGF-I+TG2; 3: TG2 control; 4: Gn control; 5: IGF-I+Gn; 6: TG2+Gn; 7: TG2+Gn+IGF-I. (Gn = glucagon)
FIGURE 5 Western blot of reaction of IGF-1R with IGF-I catalysed by TG2. The lanes from left to right are as follows: Molecular weight markers; IGF-I alone; IGF-I + IGF1R, without TG2; IGF-I + TG2; IGF1R + TG2, with no IGF-I; IGF-I + IGF1R + TG2; TG2 alone; IGF1R alone; Molecular weight markers.

### DETAILED DESCRIPTION OF THE INVENTION

### Transglutaminase protein substrates of the present invention

The present disclosure relates, in part, to regulation of insulin-like growth factor signalling pathways by modulation of an interaction between a TG and a substrate protein upon which TG exerts its enzymatic activity. The present disclosure provides in the broadest forms methods of therapy which modulate an interaction between a TG and a substrate protein described herein and methods of using this interaction as a target for finding therapeutic agents effective for the treatment of TG-associated disorders, diseases and/or conditions and in particular, cell migration and/or proliferation-associated diseases as described herein.

### Insulin-like Growth Factor

Dysregulation of the IGF signalling pathway is implicated in a plethora of clinically relevant disorders which require costly medical treatments.

To this end, elucidation of new potential targets within the IGF signalling pathway may provide hitherto unrealised opportunities for drug development. As such the present invention, in part, recognises a need for alternative targets for regulation or modification of one or more aspects of the IGF signalling pathway and thus provide potentially efficacious therapeutic agents for use in the therapy of a myriad of IGF-related diseases.

Therefore, in one form the present disclosure is broadly directed to utilisation or exploitation of the ability of IGF to be crosslinked to other proteins by way of a transglutaminase, in order to regulate or modulate the IGF signalling pathway for therapy or prevention of disease.

In a broad form, the present disclosure is predicated, at least in part, on the finding that IGF-I is a substrate for a transglutaminase activity. This finding may have potentially important implications for crosslinking of IGF-I to other proteins and in particular, crosslinking to proteins of the extracellular matrix and/or IGF-1R. It is envisaged by the inventors that modulation of an interaction between a TG and an IGF provides other avenues of regulating the clinically important IGF signalling pathway.

Furthermore, the inventors have demonstrated that TG acts to crosslink IGF-I to the IGF-I receptor, IGF-1R. This crosslinked form of a complex between IGF-I and IGF1R is postulated to be internalisable into a cell and may be an important step in IGF-I signalling.

Therefore the present disclosure provides in the broadest forms methods of therapy which modulate the IGF-TG interaction and methods of using the IGF-TG interaction as a target for finding therapeutic agents effective for the treatment of a TG-associated disease, disorder and/or condition and preferably, cell migration and/or proliferation-associated diseases as described herein.

In general aspects, the invention relates to methods of screening for and/or using the same which modulate an interaction between a TG and an IGF.

Preferably, the IGF is selected from IGF-1 and IGF-II.

Even more preferably, the IGF is IGF-I.

### Insulin-like Growth Factor Receptor Family

In another broad form, the present disclosure is directed to IGF receptor family members as substrates for a transglutaminase. Although not wishing to be bound by any particular theory, the inventors postulate that a TG-IGF receptor interaction is a control switch which determines the fate of the IGF receptors and thereby provides a trigger or control switch for a variety of different responses such as mitosis, migration or differentiation. The present disclosure is predicated, at least in part, on the finding that IGF1R is a substrate for TG2. Furthermore, TG2 is able to crosslink IGF1R to IGF-I.

The Insulin-like growth factor (IGF) receptor family (also known in the art as the insulin receptor family) comprises IGF1R (synonym: JTK13), insulin receptor, insulin receptor related receptor and insulin-IGF hybrid receptor. Preferably, the insulin-IGF hybrid receptor is insulin-IGF1 hybrid receptor. IGF receptor family members are highly similar. Members of the IGF receptor family exist as covalently bound receptor dimers at the cell surface. IGF receptors may exist as homodimers or alternatively, as hybrid receptor consisting of one-half of an IGF receptor family and one-half of a different IGF receptor family member.

In preferred embodiments, the IGF receptor family member is selected from the group consisting of IGF1R, insulin receptor, insulin receptor related receptor and insulin-IGF hybrid receptor.

More preferably, the IGF receptor is selected from the group consisting of IGF-1R, insulin receptor and an insulin-IGF hybrid receptor.

Even more preferably, the IGF receptor is selected from IGF-1R and an insulin-IGF hybrid receptor.

Yet even more preferably, the IGF receptor is IGF1R.

By *"transglutaminase"* or *"TG"* as is known in art, is meant an enzyme having, *inter alia,* the catalytic or enzymatic ability to crosslink one or more proteins through an acyl-transfer reaction between a γ-carboxamide group of a suitable protein-bound glutamine and the ε-amino group of a protein-bound lysine, which results in a ε-(γ-glutamyl)lysine isopeptide bond between a glutamine ("acyl donor") residue on one protein or peptide and a primary amine ("acyl acceptor") from a biological polyamine such as cadaverine, putrescine or spermidine, or a suitable lysine residue on another protein or peptide bond between the deprotonated lysine donor residue of one protein and the acceptor glutamine residue of another protein. A transglutaminase can also crosslink one or more proteins by way of a glutamine residue acting as a donor site. Reference is made to Griffin et al Biochem J (2002) 368: 377-396 and Lorand and Graham (2003) Nature Reviews Molecular Cell Biology 4: 140-156, which provide non-limiting examples of suitable transglutaminases (inclusive of synonyms for each transglutaminase if applicable) and are incorporated herein by reference.

Suitably, the TG of the invention is selected from the group consisting of FXIII, TG1, TG2, TG3, TG4, TG5, TG6 and TG7.

In preferred embodiments, the TG is selected from FXIII and TG2.

Even more preferably, the TG is TG2.

In particularly preferred embodiments that relate to IGF-I, the TG is TG2.

In light of the foregoing, it will be appreciated that in preferred embodiments, an interaction between a TG and its substrate protein is by way of a lysine residue and/or a glutamine residue.

In preferred embodiments which relate to IGF-I, the lysine residue is selected from the group consisting of lysine 27, lysine 65 and lysine 68 with respect to a wild-type IGF-I amino acid sequence.

Even more preferably, the lysine residue is lysine 68.

In alternative embodiments that relate to an interaction between a TG and an IGF-I by way of a TG glutamine-donor site, preferably the glutamine residue is selected from the group consisting of glutamine 15 and glutamine 40 with respect to a wild-type IGF-I amino acid sequence.

In preferred embodiments, the glutamine residue is glutamine 40.

In other preferred embodiments that relate to IGF-II, the glutamine residue and/or lysine residue is selected from glutamine 18 and lysine 65 with respect to a wild-type IGF-II amino acid sequence.

In particularly preferred embodiments that relate to an interaction between a TG and IGF-1R, the glutamine-donor site is selected from the group consisting of glutamine 14, glutamine 15, glutamine 399, glutamine 400, glutamine 287, glutamine 318, glutamine 321, glutamine 396, glutamine 511, glutamine 596, glutamine 619 and glutamine 623, with respect to a wild-type IGF-1R amino acid sequence.

In preferred embodiments that relate to IGF1R, the lysine residue is selected from the group consisting of lysine 159, lysine 191, lysine 498, lysine 530 and lysine 600, with respect to a wild-type IGF-1R amino acid sequence.

In preferred embodiments that relate to an interaction between a TG and an insulin receptor and/or a IGF-insulin hybrid receptor, the glutamine donor site is selected from the group consisting of glutamine 177 and glutamine 521, with respect to a wild-type insulin receptor amino acid sequence.

In other preferred embodiments that relate to an interaction between a TG and an insulin receptor and/or a IGF-insulin hybrid receptor, the lysine residue is selected from the group consisting of lysine 164, lysine 166, lysine 265, lysine 267, lysine 460, lysine 508, lysine 649 and lysine 652, with respect to a wild-type insulin receptor amino acid sequence.

It will be appreciated that in embodiments which contemplate an IGF-insulin hybrid receptor, an interaction with a TG may be by way of residues described hereinbefore for IGF-1R only, by way of residues hereinbefore for the insulin receptor and combinations thereof.

### Screening Methods and Therapeutic Agents

In particular aspects, the present invention is broadly directed to methods of screening, designing, engineering or otherwise producing therapeutic agents effective for the treatment of a TG-associated disease, disorder and/or condition which includes the step of modulating an interaction between a TG and a substrate protein as set out in claim 1.

It will be readily appreciated the term *"modulate ", "modulation ", "modulator"* or *"modulating"* includes within its scope an action which interferes with, prevents, disrupts, inhibits, blocks or hinders or alternatively, activates, promotes, increases or augments an interaction between a TG and an IGF and/or a TG and a member of IGF receptor family. It will be appreciated that modulators may be indirect modulators of an interaction between a TG and a substrate protein of the invention or a direct modulator of an interaction between a TG and a substrate protein of the invention.

By *"inhibition* or *"inhibit"* is meant to block, interfere, prevent, disrupt or otherwise decrease biological activity, including full blocking of the biological activity. By way of example, *"inhibition "* can refer to a decrease of about 10%, 20%, 30%, 40%, 50%, 60%, 70%, 80%, 90% or 100% in biological activity.

By *"activation* or *"activate "* is meant to promote, augment or otherwise increase biological activity. By way of example, *"activation "* can refer to an increase of about 10%, 20%, 30%, 40%, 50%, 60%, 70%, 80%, 90% or 100% in biological activity.

In preferred embodiments, the modulator is a selective modulator. In preferred embodiments, the selective modulator is a selective activator or a selective inhibitor. It is envisaged that by *"selective"* or *"selectively"* is meant that a modulator of the present invention primarily affects an interaction between a specific TG and a substrate protein of the present invention but may also have an affect on other interactions. By way of example, certain embodiments encompass situations where a modulator may selectively modulate an interaction between TG2 and IGF-I by primarily modulating TG2 but may also have an affect on other TGs such as TG1, TG3, TG4, TG5, TG6, TG7 and/or FXIII.

In preferred embodiments, the candidate agent of the invention modulates a TG-catalysed reaction to thereby modulate crosslinking of an IGF to a member of IGF receptor family and preferably, IGF-I to IGF1R. This may be by way of modulating a TG-IGF interaction or alternatively, a TG-IGF1R interaction.

Accordingly, suitable candidate agents and/or modulators of the invention may be peptides, proteins inclusive of antibodies, nucleic acids, protein complexes or other organic molecules, preferably small organic molecules, with a desired biological activity and half-life.

For the purposes of this invention, by *"isolated"* is meant material that has been removed from its natural state or otherwise been subjected to human manipulation. Isolated material may be substantially or essentially free from components that normally accompany it in its natural state, or may be manipulated so as to be in an artificial state together with components that normally accompany it in its natural state. Isolated material may be in native, chemical synthetic or recombinant form.

By "*protein*" is meant an amino acid polymer. The amino acids may be natural or non-natural amino acids, D- or L- amino acids or chemically-derivatized amino acids as are well understood in the art.

A *"polypeptide"* is a protein having fifty (50) or more amino acids.

A *"peptide* " is a protein having less than fifty (50) amino acids.

The present invention contemplates the use of and extends to protein variants which includes within their scope naturally-occurring variants such as allelic variants, orthologs and homologs and artificially created mutants, for example. Orthologs includes any mammalian ortholog of a protein inclusive of the ortholog in humans and other primates, experimental mammals such as mice, rats, hamsters and guinea pigs, mammals of commercial significance such as horses, cows, camels, pigs and sheep and also companion mammals such as dogs and cats.

In the context of the present invention, *"consisting essentially of"* or *"consist essentially of"* is meant that a polypeptide and/or peptide has one, two or no more than three amino acid residues in addition to the polypeptide and/or peptide sequence at the N- and/or C-terminus of the peptide. The additional amino acid residues may occur at one or both termini of the polypeptide and/or peptide, but is not limited thereto.

Proteins and peptides may be useful in native, chemical synthetic or recombinant synthetic form and may be produced by any means known in the art, including but not limited to, chemical synthesis, recombinant DNA technology and proteolytic cleavage to produce peptide fragments.

A recombinant protein or peptide may be conveniently prepared by a person skilled in the art using standard protocols as for example described in Sambrook et al., MOLECULAR CLONING. A Laboratory Manual (Cold Spring Harbor Press, 1989), incorporated herein by reference, in particular Sections 16 and 17; CURRENT PROTOCOLS IN MOLECULAR BIOLOGY Eds. Ausubel et al., (John Wiley & Sons, Inc. 1995-1999), incorporated herein by reference, in particular Chapters 10 and 16; and CURRENT PROTOCOLS IN PROTEIN SCIENCE Eds. Coligan et al., (John Wiley & Sons, Inc. 1995-1999) which is incorporated by reference herein, in particular Chapters 1, 5 and 6.

In those embodiments which contemplate peptides, said peptides may be in the form of peptides prepared by chemical synthesis, inclusive of solid phase and solution phase synthesis. Such methods are well known in the art, although reference is made to examples of chemical synthesis techniques as provided in Chapter 9 of SYNTHETIC VACCINES Ed. Nicholson (Blackwell Scientific Publications) and Chapter 15 of CURRENT PROTOCOLS IN PROTEIN SCIENCE Eds. Coligan et al., (John Wiley & Sons, Inc. NY USA 1995-2001). In this regard, reference is also made to International Publication WO 99/02550 and International Publication WO 97/45444.

The present invention also contemplates use of fragments of an isolated protein and/or isolated protein complex as described herein.

A *"fragment"* is a segment, domain, portion or region of a protein, or a nucleic acid encoding the same, which constitutes less than 100% of the full-length or entire protein.

A fragment preferably comprises less than 99%, 98%, 97%, 96%, 95%, 94%, 93%, 92%, 91%, 90%, 85%, 80%, 75%, 70%, 60%, 50%, 40%, 30%, 20% or as little as even 10%, 5% or 3% of the entire protein.

In particular embodiments, a fragment is a *"biologically-active fragment ".* By *"biologically-active fragment"* is meant a segment, portion or fragment of a protein, or a nucleic acid encoding same, which has at least about 0.1%, preferably at least about 10%, more preferably at least about 25% and even more preferably at least 50% of the activity of the molecule and even more preferably at least 70%, 80% or 90% of the biological activity of the entire or full length protein. In preferred embodiments, a biologically-active fragment retains retains biological, structural and/or physical activity of a full-length protein. In other preferred embodiments, a biologically-active fragment corresponds to a portion of a TG which displays a enzymatic ability to crosslink proteins, and in particular use of an IGF and/or IGF family member as a substrate. In particularly preferred embodiments, a biologically-active fragment of a TG is able to catalyse an acyl-transfer reaction between a γ-carboxamide group of a protein-bound glutamine and the ε-amino group of a protein-bound lysine, which results in a ε-(γ-glutamyl) lysine isopeptide bond between the glutamine donor residue of one protein and the acceptor primary amine from a biological polyamine or a lysine residue of another protein as hereinbefore described. In other particularly preferred embodiments, a biologically active fragment of a TG forms an isopeptide bond between a glutamine donor residue present on one or more proteins and a biological polyamine or a lysine acceptor residue.

In general embodiments, the invention contemplates a modulator which is an activator of an interaction between a TG and a substrate protein of the present invention. According to these preferred embodiments, it is envisaged that activators may be useful in promoting cell migration and/or proliferation in therapeutic treatment or facilitation of wound healing, treatment of ulcers, burns and the like and/or skin regeneration in mammals.

In other preferred embodiments, an activator may promote formation of an active state TG from an inactive TG.

In certain other preferred embodiments, the activator is an activator of a GDP-inhibited TG. According to these embodiments, the activator may remove bound GDP by acting as a competitive binding agent which mimics or closely resembles the amino acid residues which form the binding pocket of a TG and thus acts by removing the inhibitory GDP from a GDP-binding pocket of a TG. It is envisaged that ideally, that the GDP is bound into a surface pocket by a combination of charge interactions at the diphosphate and hydrophobic and Van der Waals interactions at the guanine. Therefore, such a binding pocket comprises predominantly positively charged and hydrophobic amino acid residues. A suitable non-limiting example of a GDP-binding pocket of TG comprises amino acid residues 173-174,476,478-479,482-483 and 583 of a TG2.

In light of the foregoing, it will be appreciated that an activator which may remove a bound inhibitory GDP in a TG through a competitive binding reaction has similar charge, sequence and physicochemical characteristics as the amino acid residues that form the GDP-binding pocket to thereby competitively bind and remove the inhibitory GDP. It is envisaged that such an activator is a competitive binding agent and displaces GDP from the GDP-binding pocket. Suitably, such an activator comprises predominantly positively charged amino acid residues and hydrophobic amino acid residues.

In other preferred embodiments, the activator of a GDP-inhibited TG may bind in the GDP-binding pocket, or a cleft that is revealed upon a conformational changed induced by removal of a bound GDP, to inhibit further binding of a GDP and thus possibly rendering a TG as being in a permanently active state. It is envisaged that such an activator may keep GDP displaced from a TG, and more preferably permanently replace GDP in a TG. In preferred embodiments, such an activator of a GDP-inhibited TG comprises a polyanionic amino acid sequence and more preferably, a polyanionic domain of mature VN. Even more preferably, polyanionic domain of VN is the polyanionic region corresponding to amino acids 53-64 of mature VN.

The following amino acid sequence is a segment of vitronectin that contains the aforementioned polyanionic sequence of VN. The polyanionic sequence is shown below in italics and underlines (pT=phosphothreonine, sY=sulfotyrosine) and is a very strongly negatively charged sequence:
QVTRGDVF(pT)MP*EDE(sY)(pT)V(sY)DDGEE*KNNATVH (SEQ ID NO:34)

Although not wishing to be bound by any particular theory, it is contemplated that the GDP-inhibited TG2 is held in its closed conformation by charge interactions with the diphosphate on one side, and hydrophobic/van der Waals interactions on the other In the crystal structure of active TG2 (for example, as described in Pinkas et al (2007) PLoS Biology 5; e327: 2788-2796) a cleft is present which is inaccessible in GDP-bound (inactive) TG2. This cleft is bounded by a large number of positively charged residues and has a strongly hydrophobic character at its base, in such a configuration that it appears a peptide based on a polyanionic sequence of VN bound into a TG would prevent the enzyme folding back into its inactive state.

It is envisaged that a polyanionic amino acid sequence respectively may be a component of an isolated protein complex comprising an IGF, or at least a domain of an IGF which is capable of binding a cognate IGF receptor and VN or fibronectin (FN), or at least an integrin-binding domain of VN or FN. In particularly preferred embodiments, a polyanionic amino acid sequence is a component of an isolated protein complex in the form of a synthetic chimeric protein comprising an amino acid sequence of a growth factor, or at least a domain of a growth factor which is capable of binding a cognate growth factor receptor and VN or FN, or at least an integrin-binding domain of VN or FN. It will be appreciated that in preferred embodiments, the polyanionic amino acid sequence is the polyanionic domain of mature VN.

Reference is made to International Publication No. WO/2004/069871 in the name of Queensland University of Technology which provides non-limiting examples of suitable isolated protein complexes and synthetic chimeric proteins that may be applied to the present invention

In other general embodiments, the invention provides modulators which are inhibitors of an interaction between a TG and a substrate protein of the present invention. Inhibitors are particularly suitable for use in methods of treatment in which it is desirable to inhibit or prevent cell migration and/or proliferation such as cancer cell metastasis, hyperproliferative disorders such as scarring, psoriasis and atherosclerosis.

In certain preferred embodiments, an inhibitor is a peptide that inhibits a TG catalytic activity, either reversibly or irreversibly. Such an inhibitory peptide may inhibit a TO by binding to one or more residues in the active site of a TG protein. A non-limiting example of a suitable inhibitory peptide of a TO is Ac-P(DON)LPF-NH₂ (SEQ ID NO: 1)as provided in Pinkas et al (2007) PLoS Biology, 5, e327:2788-2796.

The present invention also includes within its scope inhibitors which act upon, target or compete for a TG substrate donor site present on a TG substrate

It will be appreciated that according to these embodiments, competitive inhibitors of a TG which compete for a substrate donor site, such as a lysine-donor site, are contemplated. In preferred embodiments, such inhibitors may be small organic molecules. Suitable non-limiting example of TG inhibitors are monofunctional primary amines of the form R-NH2, where R is a linear hydrocarbon chain. A non-limiting example of a suitable primary amine inhibitor is monodansylcadaverine.

Other inhibitors contemplated by the present invention are inhibitors which are specific for a TG. In preferred embodiments, suitable TG-specific inhibitors comprise a 3-halo-4,5-dihydroisoxazole moiety. Reference is made to United States Application No. 11/213,173 (Publication No. US 2006/0052308) which provides non-limiting examples of suitable TG-specific inhibitors comprising a 3-halo-4,5-dihydroisoxazole moiety.

Although not wishing to be bound by any particular theory, a TG may be inhibited by binding of guanine nucleotides, and in particular TG may be inactivated by a bound GDP molecule and thus is in a closed GDP-bound conformation. Conversely, removal of a bound GDP from a TG may allow the TG to adopt an active conformation.

In preferred embodiments, the methods of the present invention contemplate an inhibitor that is an antibody, or an antibody fragment, which disrupts or inhibits an interaction between a TG and an IGF, or fragments thereof.

In other preferred embodiments, the methods of the present invention contemplate an inhibitor that is an antibody, or an antibody fragment, which disrupts or inhibits an interaction between a TG and an IGF receptor family member, or fragments thereof. Preferably an IGF receptor family member selected from the group consisting of IGF1R, insulin receptor, insulin receptor related receptor and insulin-IGF hybrid receptor. Even more preferably the IGF receptor is selected from the group consisting of IGF1R, insulin receptor and an insulin-IGF hybrid receptor, yet even more preferably, IGF receptor is selected from IGF1R and an insulin-IGF hybrid receptor. In particularly preferred embodiments, the IGF receptor is IGF1R.

According to certain preferred embodiments which contemplate an antibody inhibitor inhibits an interaction between a TG and an IGF receptor family member, the antibody inhibitor is suitable for use in methods of cancer treatment in which it is desirable to inhibit or prevent cell migration and/or proliferation. Such therapeutic agents are also suitable for use in treatment of autoimmune diseases such as diabetes.

In preferred embodiments, the antibody, or antibody fragment binds and/or has been raised against a transglutaminase. Reference is made to Osman et al (2002) Eur J Gastroenterol Hepatol. Nov;14(11):1217-23 which provides non-limiting examples of suitable TG antibodies for use of the present invention and methods of their production.

In other preferred embodiments, the invention contemplates an antibody, or an antibody fragment that has been raised against and/or binds to a region on an IGF and/or a member of IGF receptor family. It will be appreciated that according to certain forms of these embodiments, such an antibody or fragment thereof may render the TG substrate protein as non-crosslinkable.

As used herein, the terms *"antibody"* or *"immunoglobulin",* as used interchangeably herein, includes whole antibodies and any antigen binding fragment or single chains thereof. The terms *"antibody"* or *"immunoglobulin"* includes any antigen-binding protein product of the immunoglobulin gene complex, including immunoglobulin isotypes IgA, IgD, IgM, IgG and IgE and antigen-binding fragments thereof. Examples of antigen-binding fragments include, but are not limited to, Fab, F(ab)₂, Fv, scFv, *etc.*

It is envisaged that both polyclonal and monoclonal antibodies directed to either the entire protein or a biologically-active fragment thereof are suitable therapeutic agents.

As mentioned above, antibodies may be monoclonal or polyclonal, obtained for example by immunizing a suitable production animal (e.g. a mouse, rat, rabbit, sheep, chicken or goat). Serum or spleen cells may be then isolated from the immunized animal according to whether polyclonal or monoclonal antibodies are required.

Monoclonal antibodies may be produced by standard methods such as described in CURRENT PROTOCOLS IN IMMUNOLOGY (Eds. Coligan et al. John Wiley & Sons. 1995-2000) and Harlow, E. & Lane, D. Antibodies: A Laboratory Manual (Cold Spring Harbour, Cold Spring Harbour Laboratory, 1988). Such methods generally involve obtaining antibody-producing cells, such as spleen cells, from an animal immunized as described above, and fusing spleen cells with an immortalized fusion partner cell.

Recombinant antibodies are also contemplated. Selection of appropriate recombinant antibodies can be achieved by any of a number of methods including phage display, microarray or ribosome display, such as discussed in Hoogenboom, 2005, Nature Biotechnol. 23 1105, by way of example.

Also contemplated are antibody fragments such as Fab, F(ab)2, Fv, scFV and Fc fragments as well understood in the art.

As is also well understood in the art, in order to assist detection of antibody-antigen complexes, antibodies may be conjugated with labels including but not limited to a chromogen, a catalyst, an enzyme, a fluorophore, a chemiluminescent molecule, biotin and/or a radioisotope.

It will be appreciated by a person of skill in the art that antibodies employed for therapeutic applications in humans must have specific properties which make these antibodies suitable for use in humans. Typically, therapeutic antibodies are *"humanised ",* wherein the antibody or at least one chain thereof, typically comprises a variable framework region substantially from a human antibody and the complementary determining regions substantially from an antibody derived from a non-human (such as, but not limited to, rodent antibodies and shark antibodies). Humanised antibodies are particularly advantageous for medical applications due to the decrease likelihood of eliciting a foreign body immune reaction.

Also envisaged are antibody-like peptidomimetics in which an antibody into much smaller peptidomimetics which mimic an antibody.

Inhibitors may be in the form of a non-crosslinkable IGF or IGF receptor family member which has lost the ability to crosslink with its cognate binding protein. In preferred embodiments, the non-crosslinkable substrate protein may be mutated so TG-mediated crosslinking is abrogated. Although not wishing to be bound by any particular theory, an IGF-I mutant as described herein may bind its cognate receptor (competing with wild-type IGF-I to do so) but not be acted upon by transglutaminase and hence not trigger internalisation.

The terms *"mutant", "mutation"* and *"mutated"* are used herein generally to encompass conservative or non-conservative amino acid substitutions, deletions and/or insertions introduced into an isolated protein or fragment thereof.

It is well understood in the art that some amino acids may be changed to others with broadly similar properties without changing the nature of the activity of the protein or the structure of the protein (conservative substitutions).

Generally, non-conservative substitutions which are likely to produce the greatest changes in protein structure and function are those in which (a) a hydrophilic residue (e.g. Ser or Thr) is substituted for, or by, a hydrophobic residue (e.g. Ala, Leu, Ile, Phe or Val); (b) a cysteine or proline is substituted for, or by, any other residue; (c) a residue having an electropositive side chain (e.g. Arg, His or Lys) is substituted for, or by, an electronegative residue (e.g. Glu or Asp) or (d) a residue having a bulky hydrophobic or aromatic side chain (e.g. Val, Ile, Phe or Trp) is substituted for, or by, one having a smaller side chain (e.g. Ala, Ser) or no side chain (e.g. Gly).

With regard to mutants and/or protein variants, these can be created by mutagenising a protein or by mutagenising an encoding nucleic acid, such as by random mutagenesis, oligonucleotide-mediated (or site-directed) mutagenesis, PCR mutagenesis and cassette mutagenesis. Examples of nucleic acid mutagenesis methods are provided in Chapter 9 of CURRENT PROTOCOLS IN MOLECULAR BIOLOGY, Ausubel *et al., supra* which is incorporated herein by reference. Commercial kits are readily available such as Quick-Change site-directed mutagenesis kit from Stratagene.

It will be appreciated by the skilled person that site-directed mutagenesis is best performed where knowledge of the amino acid residues that contribute to biological activity is available. In many cases, this information is not available, or can only be inferred by molecular modelling approximations, for example.

In particularly preferred embodiments, a non-crosslinkable IGF is an isolated mutant IGF comprising a substitution of a lysine residue and/or glutamine residue which respect to a wild-type IGF amino acid sequence.

In preferred embodiments, the isolated mutant IGF comprises mutation of a lysine residue with respect to wild-type IGF amino acid sequence and/or mutation of a glutamine residue with respect to a wild-type IGF amino acid sequence. According to these embodiments, an isolated mutant IGF and preferably an isolated mutant IGF-I is an inhibitor is suitable for use in methods of treatment in which it is desirable to inhibit or prevent cell migration and/or proliferation.

In particularly preferred embodiments, the isolated mutant IGF comprises a substitution of a lysine residue with respect to wild-type IGF amino acid sequence and/or a substitution of a glutamine residue with respect to a wild-type IGF amino acid sequence.

It will be appreciated that in certain preferred embodiments, the isolated mutant IGF or isolated IGF receptor family member may comprise one or a plurality of mutations, and preferably substitutions, of one or more lysine residues and/or one or more glutamine residues. That is the invention contemplates isolated IGF mutants and isolated IGF receptor family members in which one or a plurality of lysine residues are mutated, or one or a plurality of glutamine residue are mutated or wherein a combination of lysine and glutamine residues are mutated.

In light of the foregoing, it will be appreciated that the aforementioned substitution is a conservative substitution in which the charge of the substituted residue is preserved in order to retain certain characteristics such as electrostatic topology. By way of example, in the case of a lysine residue, an appropriate amino acid substitution would be another positively-charged amino acid residue such as an arginine.

Even more preferably, the lysine residue is substituted with a residue selected from the group consisting of an alanine, an arginine residue and a histidine residue. Even more preferably, the lysine residue is substituted with an arginine residue.

In embodiments that relate to substitution of a glutamine residue, the glutamine residue is substituted with a residue selected from the group consisting of alanine, asparagine, glutamic acid and aspartic acid.

In certain embodiments that relate to an isolated IGF-1 mutant, the isolated mutant comprises one or plurality of mutations of a residue selected from the group consisting of lysine 27, lysine 65, lysine 68, glutamine 15 and glutamine 40.

In preferred embodiments that relate to an isolated IGF-I mutant, the lysine residue is selected from the group consisting of lysine 27, lysine 65 and lysine 68.

More preferably, the lysine residue is lysine 68.

In other preferred embodiments that relate to an isolated IGF-1 mutant, the glutamine residue is selected from the group consisting of glutamine 15 and glutamine 40. Preferably, the glutamine residue is glutamine 40.

In certain preferred embodiments that relate to an isolated IGF-I mutant, one or more substitutions are selected from the group consisting of:
- glutamine 15 to asparagine,
- glutamine 40 to asparagine,
- glutamine 15 to asparagine and glutamine 40 to asparagine (double mutant),
- lysine 27 to arginine,
- lysine 65 to arginine,
- lysine 68 to arginine,
- lysine 27 to arginine and lysine 65 to arginine,
- lysine 27 to arginine and lysine 68 to arginine,
- lysine 65 to arginine and lysine 68 to arginine, and
- lysine 27 to arginine, lysine 65 to arginine and lysine 68 to arginine (triple mutant).

According to other preferred embodiments, an inhibitor includes a non-crosslinkable form of an IGF receptor family member. The non-crosslinkable forms of an IGF receptor family member have particular use, although without limitation thereto, in treatment of cell proliferation disorders such as cancer. In particularly preferred embodiments, the isolated mutant IGF receptor family member comprising a mutation of a lysine residue and/or glutamine residue with respect to a wild-type IGF receptor family member amino acid sequence. Preferably the mutation is a substitution.

In embodiments which relate to an IGF-1R, the isolated mutant comprises one or a plurality of mutations of a residue selected from the group consisting of lysine 159, lysine 191, lysine 498, lysine 530 and lysine 600, glutamine 14, glutamine 15, glutamine 399, glutamine 400, glutamine 287, glutamine 318, glutamine 321, glutamine 396, glutamine 511, glutamine 596, glutamine 619 and glutamine 623, with respect to a wild-type IGF-1R sequence.

An inhibitor may be an isolated nucleic acid. The isolated nucleic acid may be an expression construct which encodes a modulator which is a protein or alternatively, the isolated nucleic acid may itself have modulator activity such as, but not limited to, an RNAi molecule or a ribozyme.

The term *"nucleic acid* as used herein designates single or double stranded mRNA, RNA, cRNA and DNA, said DNA inclusive of cDNA and genomic DNA. A nucleic acid may be native or recombinant and may comprise one or more artificial nucleotides, e.g. nucleotides not normally found in nature. RNA includes single-stranded and double-stranded unprocessed RNA, mRNA, siRNA, miRNA, RNAi and tRNA. Nucleic acid also encompasses modified purines (for example, inosine, methylinosine and methyladenosine) and modified pyrimidines (thiouridine and methylcytosine).

The term *"isolated nucleic acid"* as used herein refers to a nucleic acid subjected to *in vitro* manipulation into a form not normally found in nature. Isolated nucleic acid includes both native and recombinant (non-native) nucleic acids. For example, a nucleic acid may be isolated from human.

The terms *"mRNA", "RNA"* and *"transcript"* are used interchangeably when referring to a transcribed copy of a transcribable nucleic acid.

One particular example of an isolated nucleic acid

is an inhibitory RNA construct, such as but not limited to an inhibitory RNA that is double-stranded or otherwise comprises internal base pairing as described in more detail hereinafter. Other inhibitory RNA constructs suitable for therapy include self-cleaving RNA molecules such as ribozymes and RNA aptamers.

Inhibitory RNA constructs may include siRNA or shRNA construct that down-regulate expression of a TG protein.

A DNA construct may encode an inhibitory RNA construct.

In light of the foregoing it will be appreciated that RNA-based methods may be employed for inhibition of an interaction between a TG and an IGF and/or a member of IGF receptor family. RNA interference, and in particular (but not limited thereto) siRNA and shRNA, provides an attractive method for silencing of potential therapeutic gene targets by sequence-specific cleavage of cognate mRNA. Takeshita and Ochiya (Cancer Sci, 2006, 97: 689-696) provides numerous examples of the therapeutic potential of RNA interference against cancer

The term *"gene"* is used herein to describe a discrete nucleic acid locus, unit or region within a genome that may comprise one or more of introns, exons, splice sites, open reading frames and 5' and/or 3' non-coding regulatory sequences such as a promoter and/or a polyadenylation sequence.

Therefore a person of skill in the art will readily appreciate that the disclosure contemplates a genetic construct which comprises one or more nucleotide sequences capable of directing synthesis of an RNA molecule, said nucleotide sequence selected from the list comprising:-
(i) a nucleotide sequence transcribable to an RNA molecule comprising an RNA sequence which is substantially homologous to an RNA sequence encoded by a nucleotide sequence of interest;
(ii) a reverse complement of the nucleotide sequence of (i);
(iii) a combination of the nucleotide sequences of (i) and (ii),
(iv) multiple copies of nucleotide sequences of (i), (ii) or (iii), optionally separated by a spacer sequence;
(v) a combination of the nucleotide sequences of (i) and (ii), wherein the nucleotide sequence of (ii) represents an inverted repeat of the nucleotide sequence of (i), separated by a spacer sequence; and
(vi) a combination as described in (v), wherein the spacer sequence comprises an intron sequence spliceable from said combination.

Where the nucleotide sequence comprises an inverted repeat separated by a non-intron spacer sequence, upon transcription, the presence of the non-intron spacer sequence facilitates the formation of a stem-loop structure by virtue of the binding of the inverted repeat sequences to each other. The presence of the non-intron spacer sequence causes the transcribed RNA sequence (also referred to herein as a "transcript") so formed to remain substantially in one piece, in a form that may be referred to herein as a "hairpin". Alternatively, where the nucleotide sequence comprises an inverted repeat wherein the spacer sequence comprises an intron sequence, upon transcription, the presence of intron/exon splice junction sequences on either side of the intron sequence facilitates the removal of what would otherwise form into a loop structure. The resulting transcript comprises a double-stranded RNA (dsRNA) molecule, optionally with overhanging 3' sequences at one or both ends. Such a dsRNA transcript is referred to herein as a "perfect hairpin". The RNA molecules may comprise a single hairpin or multiple hairpins including "bulges" of single-stranded DNA occurring in regions of double-stranded DNA sequences.

Depending upon the application, the RNA molecule may be directed to a single target or alternatively, a plurality of targets.

In further general aspects, the invention relates to methods of screening for a therapeutic agent which modulates an interaction between a TG and an IGF or member of the IGF receptor family as set out in claim 1.

Particular embodiments contemplate screening, for therapeutic agents which may be a *"mimetic".* The term *"mimetic"* is used herein to refer to molecules that are designed to resemble particular functional or structural regions of proteins or peptides, and includes within its scope the terms *"agonist", "analogue "* and *"antagonist"* as are well understood in the art. Therefore in certain preferred embodiments, the therapeutic agent is an inhibitor. In other preferred embodiments, the therapeutic agent is an activator.

In one embodiment, agonists are produced which mimic an interaction between a TG and IGF or a member of the IGFR family. Such molecules may have utility as stimulators of cell migration and/or proliferation such as required for wound healing, skin regeneration and the like.

In another embodiment, antagonists are produced that prevent or inhibit the interaction between a TG and IGF or a member of the IGFR family.

Such molecules may have utility as inhibitors of cell migration and/or cell proliferation and thereby constitute useful anti-tumour agents and also in treatments such as atherosclerosis, skin disorders such as psoriasis and hypertrophic scarring that result from aberrant cell proliferation.

In certain embodiments, it may be desirable to design a mimetic such as an agent which removes GDP based upon the structural and molecular interactions of GDP bound in the GDP-binding pocket of a TG. According to these embodiments, a mimetic may be predominantly positively charged and hydrophobic in nature.

In other preferred embodiments in which a mimetic is designed to render a TG as permanently active by preventing further binding of displaced GDP, such a mimetic may be modelled on a polyanionic region corresponding to amino acids 53-64 of mature VN.

Alternatively, a mimetic may be modelled on a peptide which binds irreversibly to a residue in the active site of TG. A suitable model inhibitory peptide may be Ac-P(DON)LPF-NH₂ as described hereinbefore.

Persons skilled in the art will be aware that therapeutic agents may be identified by any number of methods as are known in the art.

Generally, therapeutic agents may be identified by way of screening libraries of molecules such as synthetic chemical libraries, including combinatorial libraries, by methods such as described in Nestler & Liu, 1998, Comb. Chem. High Throughput Screen. 1 113 and Kirkpatrick et al., 1999, Comb. Chem. High Throughput Screen 2 211.

It is also contemplated that libraries of naturally-occurring molecules may be screened by methodology such as reviewed in Kolb, 1998, Prog. Drug. Res. 51 185.

More rational approaches to designing therapeutic agents may employ X-ray crystallography, NMR spectroscopy, computer assisted screening of structural databases, computer-assisted modelling, or more traditional biophysical techniques which detect molecular binding interactions, as are well known in the art.

A review of structural bioinformatics approaches to drug discovery is provided in Fauman et al, 2003, Meth. Biochem. Anal. 44: 477.

Computer-assisted structural database searching and bioinformatic approaches are becoming increasingly utilized as a procedure for identifying and/or engineering therapeutic agents of the invention and in particular, agonist and/or antagonist molecules. Examples of database searching methods may be found in United States Patent No. 5,752,019 and International Publication WO 97/41526 (directed to identifying EPO mimetics) and United States Patents 7,158,891 and 5,680,331 which are directed to more general computational approaches to protein modelling and structural mimicry of protein activity.

Generally, other applicable methods include any of a variety of biophysical techniques which identify molecular interactions. Methods applicable to potentially useful techniques such as competitive radioligand binding assays, electrophysiology, analytical ultracentrifugation, microcalorimetry, surface plasmon resonance and optical biosensor-based methods are provided in Chapter 20 of CURRENT PROTOCOLS IN PROTEIN SCIENCE Eds. Coligan et al., (John Wiley & Sons, 1997)

A person skilled in the art will appreciate that modulating agents and in particular therapeutic agents may be in the form of a binding partner and as such, identified by interaction assays such as yeast two-hybrid approaches and the like, but without limitation thereto. Two-hybrid screening methods are provided in Chapter 20 of CURRENT PROTOCOLS IN PROTEIN SCIENCE Eds. Coligan et al., (John Wiley & Sons, 1997)

### Pharmaceutical compositions

Pharmaceutical compositions may be particularly effective or suitable for the treatment of TG-associated diseases, disorders, and/or conditions and more preferably cell migration and/or proliferation-associated diseases, disorders and/or conditions. The pharmaceutical compositions may comprise a therapeutic agent identified by any one of the aforementioned methods.

Alternatively, pharmaceutical compositions may comprise an agent selected from the group consisting of:
(i) an isolated TG, or a fragment thereof;
(ii) an isolated TG substrate, or a fragment thereof;
(iii) an isolated IGF amino acid sequence, or an analogue together with an isolated polypeptide, or a fragment thereof, that binds to or interacts with an extracellular matrix protein; and
(iv) a modulator of an interaction between a TG and an IGF.

The pharmaceutical compositions may further comprise an IGF.

The isolated TG substrate may be selected from a glutamine-donor substrate and a lysine-donor substrate. Non-limiting examples of suitable glutamine-donor substrates are NQEQVSPL (SEQ ID NO:9) and EAQQIV(SEQ ID NO:10). Furthermore, a suitable lysine-donor substrate is FKGG (SEQ ID NO:11 ), although without limitation thereto.

In a pharmaceutical composition comprising an isolated TG substrate and an IGF, the IGF may be linked to a complementary transglutaminase substrate and/or the IGF may be linked to a secondary "free" transglutaminase substrate.

In an IGF linkage to a secondary free TG substrate, said free TG substrate may be incorporated at the N-terminus of IGF preferably via a flexible linker sequence such as, but not limited to, (Gly4Ser)n and is free to interact with other proteins. International Publication No. WO04/069871 provides general examples of suitable linker sequences

Although not wishing to be bound by any particular theory, it is envisaged the N-terminal substrate sequence binds to one or more ECM protein via normal, local transglutaminase activity and thus forms a relatively permanent link to the ECM. The IGF would possibly then bind IGF-1R and be attached via its naturally-occurring TG site to form a non-internalisable complex between IGF-1R and the ECM.

When an isolated complementary TG substrate is linked to an IGF, preferably the complementary TG substrate is a glutamine donor substrate such as, but not limited to, NQEQVSPL (SEQ ID NO:9). It is envisaged that a glutamine-donor substrate may bind to a naturally-occurring TG substrate in an IGF, blocking its reactivity (but not non-covalent interaction between IGF and its cognate receptor such as IGF1R). Although not wishing to be bound by any particular theory, a result is an IGF analogue with the ability to bind the IGF1R, but not to undergo a further TG-mediated step. It will be appreciated that according to these embodiments, an isolated complementary TG substrate linked to an IGF may act as an inhibitor.

Preferably, linkage between an IGF and a complementary TG substrate occurs by way of a TG activity.

A pharmaceutical composition may include a therapeutic agent which corresponds to an IGF-containing complex which is unable to be internalised into a cell. In preferred embodiments, such a non-internalisable IGF-containing complex or therapeutic agent may comprise an IGF together with an isolated polypeptide, or a fragment thereof, that binds to or interacts with an extracellular matrix protein. A non-limiting example of a suitable isolated polypeptide is an isolated polypeptide comprising a collagen-binding sequence from FN. It is envisaged, although without being bound by a particular theory, that permanently attaching IGF-I to said isolated polypeptide may bind the integrin-TG2-IGF1R complex together at the point of attachment of IGF-I, and attach this complex to the extracellular collagen matrix, thus inhibiting internalisation of said complex. Alternatively a non-internalisable IGF can take the form of an IGF that may be attached via the N-terminus to a particle that are small enough to leave the bloodstream and lodge in tissues, but too big to be internalised into a cell (0.5-10 microns or so). The

IGF would be bound to a synthetic particle which is too big to internalise. It is envisaged that the IGF may be attached via metal affinity using a His-tag, or native chemical ligation using an *N*-terminal cysteine.

Suitably, pharmaceutical compositions further comprise a pharmaceutically acceptable carrier, diluent or excipient.

By *"pharmaceutically-acceptable carrier, diluent or excipient"* is meant a solid or liquid filler, diluent or encapsulating substance that may be safely used in systemic administration. Depending upon the particular route of administration, a variety of carriers, well known in the art may be used. These carriers may be selected from a group including sugars, starches, cellulose and its derivatives, malt, gelatine, talc, calcium sulfate, vegetable oils, synthetic oils, polyols, alginic acid, phosphate buffered solutions, emulsifiers, isotonic saline and salts such as mineral acid salts including hydrochlorides, bromides and sulfates, organic acids such as acetates, propionates and malonates and pyrogen-free water.

A useful reference describing pharmaceutically acceptable carriers, diluents and excipients is Remington's Pharmaceutical Sciences (Mack Publishing Co. N.J. USA, 1991) which is incorporated herein by reference.

Any safe route of administration may be employed for providing a patient with the composition of the invention. For example, oral, rectal, parenteral, sublingual, buccal, intravenous, intra-articular, intra-muscular, intra-dermal, subcutaneous, inhalational, intraocular, intraperitoneal, intracerebroventricular, transdermal and the like may be employed. Intra-muscular and subcutaneous injection is appropriate, for example, for administration of immunotherapeutic compositions, proteinaceous vaccines and nucleic acid vaccines. In the case of gene therapy, which contemplates the use of electroporation or liposomal transfection into tissues, the drug may be transfected into cells together with the DNA.

Dosage forms include tablets, dispersions, suspensions, injections, solutions, syrups, troches, capsules, suppositories, aerosols, transdermal patches and the like. These dosage forms may also include injecting or implanting controlled releasing devices designed specifically for this purpose or other forms of implants modified to act additionally in this fashion. Controlled release of the therapeutic agent may be effected by coating the same, for example, with hydrophobic polymers including acrylic resins, waxes, higher aliphatic alcohols, polylactic and polyglycolic acids and certain cellulose derivatives such as hydroxypropylmethyl cellulose. In addition, the controlled release may be effected by using other polymer matrices, liposomes and/or microspheres.

Compositions suitable for oral or parenteral administration may be presented as discrete units such as capsules, sachets or tablets each containing a pre-determined amount of one or more therapeutic agents of the invention, as a powder or granules or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association one or more agents as described above with the carrier which constitutes one or more necessary ingredients. In general, the compositions are prepared by uniformly and intimately admixing the agents with liquid carriers or finely divided solid carriers or both, and then, if necessary, shaping the product into the desired presentation.

The above compositions may be administered in a manner compatible with the dosage formulation, and in such amount as is pharmaceutically-effective. The dose administered to a patient, should be sufficient to effect a beneficial response in a patient over an appropriate period of time. The quantity of agent(s) to be administered may depend on the subject to be treated inclusive of the age, sex, weight and general health condition thereof, factors that will depend on the judgement of the practitioner.

### Therapeutic Methods

Methods of treating a TG-associated disease, disorder and/or condition may comprise administering a therapeutic agent effective for the treatment of a transglutaminase-associated disorder, disease and/or condition and/or a pharmaceutical composition as described herein.

Preferably, the TG-associated disease, disorder and/or condition is a cell migration and/or proliferation-associated disorder, disease and/or condition.

The cell migration and/or proliferation-associated disorder, disease and/or condition may be selected from the group consisting of wound healing, psoriasis, atherosclerosis, cancer, tumour resistance to chemotherapy, a burn, an ulcer and hypertrophic scarring.

The transglutaminase-associated disorder, disease and/or condition may be an autoimmune disease.

The autoimmune disease may be diabetes and even more preferably, type I diabetes.

It will also be appreciated that treatment methods and pharmaceutical compositions may be applicable to prophylactic or therapeutic treatment of mammals, inclusive of humans and non-human mammals such as livestock (e.g. horses, cattle and sheep), companion animals (e.g. dogs and cats), laboratory animals (*e.g.* mice rats and guinea pigs) and performance animals (*e.g* racehorses, greyhounds and camels), and animals used as a source of cells, organs and tissues for xenotransplantation, although without limitation thereto.

Methods of cosmetic treatment may comprise administering the therapeutic agents and/or pharmaceutical compositions to improve or enhance skin quality or skin appearance.

Such treatments may include prevention or remedediation of skin disorders such as psoriasis and hypertrophic scarring that result from aberrant skin cell proliferation.

Alternatively, methods of treatment are contemplated whereby tumour metastasis is prevented or inhibited by blocking tumour cell migration to a secondary site. In addition, methods of treating cancer by blocking cell proliferation also contemplated.

Also contemplated are methods which modulate an interaction between a TG and an IGF in treatment of IGF-mediated or associated tumour resistance to chemotherapy.

Also contemplated are methods which modulate an interaction between a TG and an IGF-1R in treatment of IGF-1R-mediated or associated tumour resistance to chemotherapy.

It will be understood that the methods of promoting cell migration and/or proliferation as described herein may also relate to *in vitro* or *ex vivo* cell culture techniques as well as prophylactic and/or therapeutic methods of treatment of an animal.

So that the invention may be readily understood and put into practical effect, the following non-limiting Examples are provided.

### EXAMPLES

### Example 1

### IGF-I Contains a site for crosslinking by transglutaminase enzymes

In humans, the transglutaminases (TGs) are a family of nine closely related enzymes, only five of which have been studied in any detail. Those studied to date share very similar substrate preferences, and homology studies suggest that the remaining four are no exception. Rather, the TGs differ mostly in their temporal and spatial expression, and their mechanisms of activation.

The most common role of TGs is the catalysis of isopeptide bond formation between selected glutamine and lysine residues, with the concomitant release of ammonium. However, TGs have also been found to catalyse the hydrolytic deamidation of glutamine residues, as well as, more recently, the partial reversal of the crosslinking reaction via hydrolysis to glutamic acid and lysine residues.

TG2, or tissue TG, is the most well-characterised of the TGs after factor XIII. It is ubiquitous within the body, and is found in large quantities within the cytoplasm, in the extracellular matrix (ECM) and at the cell surface. Extracellular TG2 is inactive under normal circumstances, but becomes activated immediately upon tissue damage.

In the present experiments, IGF-I was incubated with FXIII and PEG functionalized with either a glutamine (Q)- or lysine (K)-donor TG substrate peptide. A Western Blot using a monoclonal antibody against IGF-I (Figure 1a) clearly showed that after 1h IGF-I was quantitatively incorporated into the Q-donor, but not the K-donor PEG, indicating that IGF-I contains a TG K-donor site.

The-C-terminal "D domain" of IGF-I is probably very flexible in solution, has the following amino acid sequence -⁶¹CAPLKPAKSA⁷⁰-OH (SEQ ID NO:12). It is the present inventors view that this D domain represents a hitherto unknown site for the action of TGs at K⁶⁸, leading to covalent incorporation of IGF-I into the ECM and/or blood clots.

In order to confirm this finding, we repeated this experiment using TG2 rather than FXIII, and the known TG2 Gln-donor substrate glucagon (Gn) as a probe (Figure 4). To our surprise, this reaction (lane 7) produced a wide range of molecular weight species, indicating the formation of higher-order IGF-I-Gn polymers. Similarly, IGF-I alone when reacted with TG2 (lane 2) produced a series of bands corresponding to IGF-I oligomers. This was not seen for FXIII (not shown) and demonstrates that IGF-I contains a Gln-donor substrate for TG2 (but not FXIII). The large band >75kDa in lane 2 is apparently due to the formation of covalent complexes between IGF-I and TG2 itself; polymerization of IGF-I is also evident. Reaction of IGF-I with Gn (lane 7) also produced polymeric species. A band of MW consistent with (IGF-I)₁Gn₁ is indicated by an arrow.

Based on these preliminary findings, the present inventors propose a number of major modifications to the current model of the IGF-I signalling pathway, which are summarised in Figure 2. **Step 1**: Under homeostatic conditions, integrin-bound to ECM (eg fibronectin), TG2 inactive, receptor free or bound to immobilised growth factor. **Step 2:** Vitronectin displaces fibronectin and activates TG2 **Step 3:** TG links receptor to transport mediator **Step 4:** Transport via lipid raft to clathrin-coated pit **Step 5: In** early endosome, the identity of the transport mediator determines destination and hence nature of signalling.

### STRUCTURAL STUDIES OF ASSOCIATED PROTEINS

IGF-I is known to bind in a pocket bounded by the three *N*-terminal domains of the IGF1R. Epa and Ward have produced a computational 3D model of this complex to fit all currently known information about this interaction.¹ Intriguingly, in this configuration the D domain Lys residues are exposed and appear to have a range of mobility that brings them very close to Gln 14 and 15 on the IGF1R *N*-terminal domain (Figure 3b). Comparison of known sequences on the ExPASy proteomics server (Figure 3a) indicate that these residues appeared in the mammalian IGF1R subsequent to its divergence from the IR - and, indeed, subsequent to the divergence of mammals from birds. The present inventors are of the view that the evolutionarily novel residues indicates that the IGF1R may be crosslinked to IGF-I by a TG as an essential step in IGF-I signalling in mammals.

### ROLE OF MATRIX-BOUND IGF-I ISOFORMS

The IGF-I gene gives rise to at least three splice variants with identical sequence in residues 1-70, but with different, somewhat tissue-specific "E domain" peptides at the C terminus.¹¹ The dominant species found in circulation is IGF-I produced in the liver; IGF-I isoforms produced in other tissues tend to remain in the tissue of origin.⁶ The mechanisms behind this retention are unclear.

The splice variant proIGF-IA has been found to be secreted in *in vitro* culture of human hepatoma and B lymphocytes, while proIGF-IC, dubbed "mechano growth factor" has been found to be secreted by skeletal muscle cells in response to stretching or damage, and is a potent stimulator of muscle hypertrophy,³⁴ apparently acting on satellite cells recruited to the site of damage.¹¹ Processing of the proIGF-I to mature IGF-I is thought to be mediated by cleavage at R71 by serine proteases; mutational studies have shown that K68 is required for this cleavage to occur. The present inventors are of the view that TG-catalysed cross-linking of proIGF-I may preserve the bioactive E-domain peptide within the ECM.

### ROLE OF TG2 IN IGF-I MEDIATED IGF1R INTERNALIZATION AND RECYCLING

It is the present inventors' view that stimulation of the IGF1R by matrix-bound, non-internalizable IGF-I is involved in tissue homeostasis, while TG-mediated internalization of IGF-I from the bloodstream is involved in initiation and continuation of the wound-healing response, and the very similar behaviour observed in malignant cancers.

However, the majority of IGF1R internalized in this fashion may need to be recycled back to the cell surface in an active form. Therefore the present inventors postulate that the TG-mediated crosslink between IGF-I and the IGF1R would need to be reversed to yield the original Lys and Gln residues - a phenomenon that has not been reported to date. However, it is the present inventors view that TG-formed isopeptide bonds may be cleaved by TG2 within endosomes.

### Example 2

The sequence PEDE(sY)(pT)V(sY)DDGEEKNNATVH (SEQ ID NO:13), where sY is sulfotyrosine and pT is phosphothreonine, from the polyanionic sequence of VN was docked against the crystal structure of active TG2 (PDB ID 2Q3Z) using Autodock Vina as described for Figure 4 above. The results are shown in Figure 7 where the lowest-energy binding mode of the peptide is shown. This binds with a calculated energy of 32 kJ/mol into a positively charged cleft which is occluded in the GDP-bound inactive TG2. Binding in this fashion would prevent the TG2 returning to its inactive state.

### Example 3

### IGF1R as a substrate for TG2

TG2 (0.70 µM) was reacted with IGF-I (25 µg/mL) and/or recombinant extracellular domain of IGF1R (25 µg/mL; commercially available from R&D Systems) for 1 hour in the presence of 1mM CaCl₂, pH 7.5. At this point, the reaction was stopped by the addition of SDS-PAGE loading buffer. Samples were run through a 4%-12% SDS-PAGE gel, transferred to a PVDF membrane and probed for IGF-I using a goat anti-human IGF-I primary (1:5000 dilution) and HRP-conjugated rabbit anti-goat secondary (1:10,000), and visualised using chemiluminescence.

The rationale for this assay was to specifically test whether TG2 will crosslink IGF-I to the IGF1R and therefore demonstrate that IGF1R is a TG substrate. For it to be able to do this, both IGF-I and the IGF1R would have to be transglutaminase substrates. Because TG2 catalyses the formation of an isopeptide bond which is essentially as stable as a peptide backbone bond, the complex formed will not be broken apart under reducing SDS-PAGE conditions (whereas disulfide and non-covalent interactions will be broken).

So, in this assay we reacted IGF-I and the soluble recombinant extracellular portion of the receptor with TG2, and probed for where IGF-I ended up using a standard Western blot approach. The "smoking gun" for a reaction would be staining for IGF-I appearing at a molecular weight corresponding to the IGF1R or one of its fragments.

IGF1R that was used in this assay breaks up into four bands in reducing SDS-PAGE: 35, 40, 125 and 160 kDa, corresponding (in order) to two variants of the beta domain, the alpha domain, and an improperly-processed species where the alpha and beta domains were never proteolytically cleaved from each other during production. All known binding sites for IGF-I are on the alpha domain of the receptor, so that's where we expected to see it attach.

In reference to Figure 5, the lanes are, in order from left to right as appearing on the figure:
Molecular weight markers
IGF-I alone (gives a band at around 8kDa)
IGF-I + IGF1R, without TG2 (expect to see only the IGF-I band)
IGF-I + TG2 (gives a mess of IGF-I polymers)
IGF1R + TG2, with no IGF-I (don't expect to see anything)
IGF-I + IGF1R + TG2 (if TG2 attaches IGF-I to the receptor, expect to see bands appear at molecular weights corresponding to the receptor.)
TG2 alone (don't expect to see anything)
IGF1R alone (don't expect to see anything)
Molecular weight markers

Accordingly, what we see in the IGF-I + IGF1R + TG2 lane is a set of bands which don't appear in any other lanes, and which correspond to the molecular weight of the alpha domain, the uncleaved alpha-beta construct and (tentatively) a receptor dimer. This is a positive demonstration that TG2 recognises sites on both the IGF1R and IGF-I, and is able to bind the two together.

### Example 4

### Locate the TG site in IGF-I, and in IGF-II

The above data indicates that IGF-I contains a lysine-donor TG site, IGF-I has three lysine residues in K68, K27 and K65, which are potential candidates for a lysine-donor TG sites. In order to ascertain the role of each lysine residue in crosslinking, the following experiments will be undertaken.

Recombinant IGF-I and IGF-II will be reacted with FXIII or TG2 and a synthetic, biotinylated glutamine-donor peptide, NQEQVSPLK(biotin)-OH (SEQ ID NO:14), similar to that used in the hydrogel system of Ehrbar *et al.* ^{14, 15} Following trypsin digestion of the reaction mixture, the biotinylated fragments will, if necessary, be purified by streptavidin affinity and identified by matrix-assisted laser desorption/ionization mass spectrometry (MALDI-MS) and, if necessary, N-terminal sequencing.

In addition, to further demonstrate the specific lysine-donor TG site, a series of IGF-I analogues will be generated. This will involve site-directed mutagenesis and substitution of the 3 lysine residues of IGF-I to arginines, conservative mutations which will minimise changes to the electrostatic topology, while rendering the mutated residues unreactive towards TG. This will involve the production of K27R IGF-I, K65R IGF-I and K68R IGF-I. Expression and purification of the recombinant IGF analogues will use procedures that have been established and validated for the production of cGMP-grade IGF-I. Reactivity of each IGF-I analogue with the biotinylated glutamine-donor peptide will be assessed against rIGF-I using the approach described above.

### Investigate the activity of TGs under conditions similar to the endosomal environment

In order to assess, in part, whether TG2 mediates the activation, endocytosis and recycling of the IGF1R by repeated attachment and release of IGF-I at Gln 14 or 15, the following experiments will be performed to determine TG2-mediated crosslinking under acidic conditions similar to the endosomal environment.

IGF-I-Gln substrate conjugates will be prepared using FXIII or TG2. Once the reaction is complete, aliquots will be adjusted to pH values ranging from 5 to 8. Cleavage of the IGF-I from the Gln substrate will be monitored by SDS-PAGE and Western Blotting and/or reverse phase high performance liquid chromatography (HPLC).

In order to test whether hydrolysed Gln substrates remain reactive towards TGs, synthetic Gln substrate analogues will be commissioned with the active Gln replaced by Glu. These will be screened for activity against IGF-I via standard Western Blot techniques as described above.

In a complementary approach, synthetic peptide analogues of TG-crosslinked substrates functionalised with a fluorophore and quencher suitable for Förster resonance energy transfer (FRET) analysis¹⁷ will be commissioned from Mimotopes. Cleavage of the substrates by TGs will be monitored in real time using a fluorescence microplate reader. Repeated cycling of pH between ∼6 and ∼7.4 will allow us to directly test our hypothesis that TG crosslinking is a reversible cycle.

### Explore the role of the TG site in IGF signalling

An IGF-I mutant with a K→R substitution at the TG site will be tested for activity in model cell cultures in competitive assays against wild-type IGF-I. Cultures stimulated with various concentrations of IGF-I and ^{K→R}IGF-I will be analysed for relative proliferation and migration via standard protocols.

### Explore the effects of immobilized IGFs and derivatives on cell cultures

The high reactivity of IGF-I towards FXIII observed in the above experiments strongly suggests that matrix-bound IGF(s) have biological signalling roles that are distinct from the soluble form. Thus, preliminary experiments looking at the behaviour of cell types of interest when exposed to matrix-bound IGFs and their derivatives will be performed.

The TG-crosslinked hydrogel system developed and currently used by Ehrbar *et al.*^{14, 15} and further developed by the present inventors represents an ideal platform for the testing of such activity, as it allows cells to be conveniently and mildly encapsulated within a degradable structure whose make-up is entirely defined by the experimenter. IGF-I, IGF-II, chimeric VN:IGF-I constructs and, if possible, proIGF-I variants will be incorporated into hydrogels with and without added integrin-binding RGD peptides. The effects of these proteins on at least wound healing, on the proliferation and migration of dermal fibroblasts will be assessed.

### Conclusions

The IGF signalling network, despite being one of the first and most studied biochemical systems, has many aspects which are as yet not fully understood. In particular, the D domain has been a point of contention in the literature for many years since, despite being highly conserved between distantly related species, no definitive role for it in IGF-I signalling has been identified.

With observed roles in many different (patho)physiological systems, the IGF-I axis is also a popular target for drug development. The present inventors observation that IGF-I has a specific site for incorporation into the ECM opens a new avenue of investigation, and may lead to a significant shift in the understanding of how IGF-I regulates the wound healing response.

### REFERENCES

1. Epa, V.C. and C.W. Ward, Model for the complex between the insulin-like growth factor I and its receptor: towards designing antagonists for the IGF-I receptor. Protein Eng. Des. Sel., 2006. 19(8): 377-384.
2. Liu, S., et al., Structural basis for the guanine nucleotide-binding activity of tissue transglutaminase and its regulation of transamidation activity. Proc. Natl. Acad. Sci. U. S. A., 2002. 99(5): 2743-2747.
3. Denley, A., et al., Molecular interactions of the IGF system. Cytokine Growth Factor. Rev., 2005.16(4-5): 421-439.
4. Adams, G.R., Exercise Effects on Muscle Insulin Signaling and Action: Invited Review: Autocrinelparacrine IGF-I and skeletal muscle adaptation. J Appl Physiol, 2002. 93(3): 1159-1167.
5. Goldspink, G., Changes in muscle mass and phenotype and the expression of autocrine and systemic growth factors by muscle in response to stretch and overload. J. Anat., 1999. 194(3): 323-334.
6. Dobrowolny, G., et al., Muscle Expression of a Local Igf-1 Isoform Protects Motor Neurons in an ALS Mouse Model. J. Cell Biol., 2005. 168(2): 193-199.
7. Hyde, C., et al., Insulin-like Growth Factors (IGF) and IGF-Binding Proteins Bound to Vitronectin Enhance Keratinocyte Protein Synthesis and Migration. J Investig Dermatol, 2004. 122(5): 1198-1206.
8. Samani, A.A., et al., The Role of the IGF System in Cancer Growth and Metastasis: Overview and Recent Insights. Endocr Rev, 2007. 28(1): 20-47.
9. Bayes-Genis, A., et al., The Insulin-Like Growth Factor Axis : A Review of Atherosclerosis and Restenosis. Circ Res, 2000. 86(2): 125-130.
10. Blakytny, R., et al., Lack of insulin-like growth factor 1 (IGF1) in the basal keratinocyte layer of diabetic skin and diabetic foot ulcers. J. Pathol., 2000. 190(5): 589-594.
11. Barton, E.R., The ABCs of IGF-I isoforms: impact on muscle hypertrophy and implications for repair. Appl. Physiol. Nutr. Metab., 2006. 31: 791-797.
12. Upton, Z., et al., Vitronectin: Growth Factor Complexes Hold Potential as a Wound Therapy Approach. J Invest Dermatol, 2008. 128(6): 1535-1544. 13. Van Lonkhuyzen, D.R., et al., Chimeric vitronectin:insulin-like growth factor proteins enhance cell growth and migration through co-activation of receptors. Growth Factors, 2007. 25(5): 295 - 308.
14. Ehrbar, M., et al., Biomolecular hydrogels formed and degraded via site-specific enzymatic reactions. Biomacromolecules, 2007. 8(10): 3000-3007.
15. Ehrbar, M., et al., Enzymatic formation of modular cell-instructive fibrin analogs for tissue engineering. Biomaterials, 2007. 28(26): 3856-3866.

## Claims

1. A method of screening for a therapeutic agent effective for the treatment of a transglutaminase (TG)-associated disorder, disease and/or condition, said method including the step of determining whether a candidate agent modulates the interaction between:
a TG and an insulin-like growth factor (IGF) or a TG and a member of the IGF receptor family,
to thereby determine whether the candidate agent serves as a modulator of TG mediated crosslinking, and is a therapeutic agent effective for the treatment of a TG-associated disorder, disease and/or condition,
wherein the TG is selected from the group consisting of FXIII, TG1, TG2, TG3, TG4, TG5, TG6 and TG7, and
wherein the member of the IGF receptor family is selected from the group consisting of IGF-1R, insulin receptor, insulin receptor related receptor and insulin-IGF hybrid receptor.

2. The method of claim 1, wherein the candidate agent is a modulator selected from the group consisting of an isolated peptide, an isolated polypeptide, an antibody, an isolated nucleic acid and a small organic molecule.

3. The method of claim 2, wherein the modulator modulates a substrate donor site for a TG selected from an acyl-donor substrate and an acyl-acceptor substrate.

4. The method of claim 3, wherein the substrate donor site comprises a residue selected from a lysine and a glutamine.

5. The method of claim 1, wherein the candidate agent is a selective modulator.

6. The method of claim 5, wherein the selective modulator is selected from an activator and an inhibitor.

7. The method of claim 6, wherein the activator is an activator of a GDP-inhibited TG or;
wherein the inhibitor is a TG specific inhibitor, optionally wherein the TG specific inhibitor comprises a 3-halo-4, 5-dihydroisoxazole moiety.

8. The method according to claim 7, wherein the activator is selected from an isolated peptide, an isolated polypeptide and an isolated protein complex, comprising a polyanionic amino acid sequence which is a polyanionic domain of VN corresponding to amino acids 53-64 of mature VN.

9. The method according to claim 6, wherein the inhibitor is selected from the group consisting of an antibody, an isolated nucleic acid, a non-crosslinkable isolated IGF, a non-crosslinkable member of IGF receptor family, a TG inhibitor, an inhibitor of a substrate donor site of a TG and a competitive inhibitor of a substrate donor site of a TG.

10. The method of claim 9, wherein the non-crosslinkable isolated IGF is an isolated mutant IGF comprising a mutation of a lysine residue and/or a glutamine residue with respect to a wild-type IGF amino acid sequence.

11. The method of claim 10, wherein the isolated mutant IGF is an isolated IGF-I mutant, said isolated IGF-I mutant comprises one or a plurality of mutation/s of a residue selected from the group consisting of lysine 27, lysine 65, lysine 68, glutamine 15 and glutamine 40, with respect to a wild-type IGF-I amino acid sequence.

12. The method of claim 9, wherein the non-crosslinkable IGF receptor family member is an isolated IGF receptor family member mutant comprising a mutation of a lysine residue and/or a glutamine residue with respect to a wild-type IGF receptor family member amino acid sequence or;
wherein the non-crosslinkable IGF receptor family member is an isolated IGF1R mutant comprising a mutation of a lysine residue and/or a glutamine residue.

13. The method of claim 12, wherein the isolated IGF1R mutant comprises one or a plurality of mutation/s of a residue selected from the group consisting of lysine 159, lysine 191, lysine 498, lysine 530 and lysine 600, glutamine 14, glutamine 15, glutamine 399, glutamine 400, glutamine 287, glutamine 318, glutamine 321, glutamine 396, glutamine 511, glutamine 596, glutamine 619 and glutamine 623, with respect to a wild-type IGF-1R sequence.

## Patentansprüche

1. Verfahren zum Screenen auf einen therapeutischen Wirkstoff, der zur Behandlung einer/s Transglutaminase- (TG-) assoziierten Störung, Erkrankung und/oder Leidens wirksam ist, wobei das Verfahren den Schritt des Bestimmens umfasst, ob ein Wirkstoffkandidat die Wechselwirkung zwischen
einer TG und einem insulinähnlichen Wachstumsfaktor (IGF) oder einer TG und einem Mitglied der IGF-Rezeptorfamilie moduliert,
um so zu bestimmen, ob der Wirkstoffkandidat als Modulator von TG-vermittelter Vernetzung dient und ein therapeutischer Wirkstoff ist, der zur Behandlung einer/s TG-assoziierten Störung, Erkrankung und/oder Leidens wirksam ist,
wobei die TG aus der aus FXIII, TG1, TG2, TG3, TG4, TG5, TG6 und TG7 bestehenden Gruppe ausgewählt ist und
wobei das Mitglied der IGF-Rezeptorfamilie aus der aus IGF-1R, einem Insulinrezeptor, einem mit dem Insulinrezeptor verwandten Rezeptor und einem Insulin-IGF-Hybridrezeptor bestehenden Gruppe ausgewählt ist.

2. Verfahren nach Anspruch 1, wobei der Wirkstoffkandidat ein Modulator ist, der aus der aus einem isolierten Peptid, einem isolierten Polypeptid, einem Antikörper, einer isolierten Nucleinsäure und einem kleinen organischen Molekül bestehenden Gruppe ausgewählt ist.

3. Verfahren nach Anspruch 2, wobei der Modulator eine Substratdonorstelle für eine TG moduliert, die aus einem Acyldonorsubstrat und einem Acylakzeptorsubstrat ausgewählt ist.

4. Verfahren nach Anspruch 3, wobei die Substratdonorstelle einen Rest umfasst, der aus einem Lysin und einem Glutamin ausgewählt ist.

5. Verfahren nach Anspruch 1, wobei der Wirkstoffkandidat ein selektiver Modulator ist.

6. Verfahren nach Anspruch 5, wobei der selektive Modulator aus einem Aktivator und einem Inhibitor ausgewählt ist.

7. Verfahren nach Anspruch 6, wobei der Aktivator ein Aktivator einer GDP-inhibierten TG ist oder
wobei der Inhibitor ein TG-spezifischer Inhibitor ist, wobei der TG-spezifische Inhibitor gegebenenfalls eine 3-Halogen-4,5-dihydroisoxazol-Gruppierung umfasst.

8. Verfahren nach Anspruch 7, wobei der Aktivator aus einem isolierten Peptid, einem isolierten Polypeptid und einem isolierten Proteinkomplex, der eine polyanionische Aminosäuresequenz umfasst, die eine polyanionische Domäne von VN ist, die den Aminosäuren 53-64 von reifem VN entspricht, ausgewählt ist.

9. Verfahren nach Anspruch 6, wobei der Inhibitor aus der aus einem Antikörper, einer isolierten Nucleinsäure, einem nicht vernetzbaren, isolierten IGF, einem nicht vernetzbaren Mitglied der IGF-Rezeptorfamilie, einem TG-Inhibitor, einem Inhibitor einer Substratdonorstelle einer TG und einem kompetitiven Inhibitor einer Substratdonorstelle einer TG bestehenden Gruppe ausgewählt ist.

10. Verfahren nach Anspruch 9, wobei der nicht vernetzbare, isolierte IGF eine isolierte IGF-Mutante ist, die eine Mutation eines Lysinrests und/oder eines Glutaminrests in Bezug auf eine Wildtyp-IGF-Aminosäuresequenz umfasst.

11. Verfahren nach Anspruch 10, wobei die isolierte IGF-Mutante eine isolierte IGF-I-Mutante ist, wobei die isolierte IGF-I-Mutante eine oder eine Vielzahl von Mutationen eines Rests, der aus der aus Lysin 27, Lysin 65, Lysin 68, Glutamin 15 und Glutamin 40 bestehenden Gruppe ausgewählt ist, in Bezug auf eine Wildtyp-IGF-I-Ami-nosäuresequenz umfasst.

12. Verfahren nach Anspruch 9, wobei das nicht vernetzbare IGF-Rezeptorfamilien-mitglied eine isolierte IGF-Rezeptorfamilienmitgliedmutante ist, die eine Mutation eines Lysinrests und/oder eines Glutaminrests in Bezug auf eine Wildtyp-IGF-Rezep-torfamilienmitglied-Aminosäuresequenz umfasst; oder
wobei das nicht vernetzbare IGF-Rezeptorfamilienmitglied eine isolierte IGF1R-Mutante ist, die eine Mutation eines Lysinrests und/oder eines Glutaminrests umfasst.

13. Verfahren nach Anspruch 12, wobei die isolierte IGF1 R-Mutante eine oder eine Vielzahl von Mutationen eines Rests, der aus der aus Lysin 159, Lysin 191, Lysin 498, Lysin 530 und Lysin 600, Glutamin 14, Glutamin 15, Glutamin 399, Glutamin 400, Glutamin 287, Glutamin 318, Glutamin 321, Glutamin 396, Glutamin 511, Glutamin 596, Glutamin 619 und Glutamin 623 ausgewählt ist, in Bezug auf eine Wildtyp-IGF-1 R-Sequenz umfasst.

## Revendications

1. Procédé de criblage d'un agent thérapeutique efficace pour le traitement d'un trouble, une maladie et/ou une affection associé(e) à la transglutaminase (TG), ledit procédé comprenant l'étape consistant à déterminer si un agent candidat module l'interaction entre :
une TG et un facteur de croissance insulinomimétique (IGF) ou un TG et un membre de la famille de récepteurs d'IGF,
de manière à déterminer si l'agent candidat sert de modulateur de la réticulation médiée par TG, et est un agent thérapeutique efficace pour le traitement d'un un trouble, une maladie et/ou une affection associé(e) à TG,
dans lequel la TG est choisie dans le groupe constitué de FXIII, TG1, TG2, TG3, TG4, TG5, TG6 et TG7, et
dans lequel le membre de la famille de récepteurs d'IGF est choisi dans le groupe constitué d'IGF-1R, un récepteur d'insuline, un récepteur apparenté au récepteur d'insuline et un récepteur hybride d'insuline-IGF.

2. Procédé de la revendication 1, dans lequel l'agent candidat est un modulateur choisi dans le groupe constitué d'un peptide isolé, un polypeptide isolé, un anticorps, un acide nucléique isolé et une petite molécule organique.

3. Procédé de la revendication 2, dans lequel le modulateur module un site donneur de substrat pour un TG choisi parmi un substrat donneur d'acyle et un substrat accepteur d'acyle.

4. Procédé de la revendication 3, dans lequel le site donneur de substrat comprend un résidu choisi parmi une lysine et une glutamine.

5. Procédé de la revendication 1, dans lequel l'agent candidat est un modulateur sélectif.

6. Procédé de la revendication 5, dans lequel le modulateur sélectif est choisi parmi un activateur et un inhibiteur.

7. Procédé de la revendication 6, dans lequel l'activateur est un activateur d'une TG inhibée par GDP ou ;
dans lequel l'inhibiteur est un inhibiteur spécifique de TG, facultativement dans lequel l'inhibiteur spécifique de TG comprend un fragment 3-halogéno-4, 5-dihydroisoxazole.

8. Procédé selon la revendication 7, dans lequel l'activateur est choisi parmi un peptide isolé, un polypeptide et un complexe de protéine isolé, comprenant une séquence d'acides aminés polyanionique qui est un domaine polyanionique de VN correspondant aux acides aminés 53-64 de VN mature.

9. Procédé selon la revendication 6, dans lequel l'inhibiteur est choisi dans le groupe constitué d'un anticorps, un acide nucléique isolé, un IGF isolé non réticulable, un membre non réticulable de la famille de récepteurs d'IGF, un inhibiteur de TG, un inhibiteur d'un site donneur de substrat d'une TG et un inhibiteur compétitif d'un site donneur de substrat d'une TG.

10. Procédé de la revendication 9, dans lequel l'IGF isolé non réticulable est un IGF mutant isolé comprenant une mutation d'un résidu lysine et/ou d'un résidu glutamine par rapport à une séquence d'acides aminés d'IGF de type sauvage.

11. Procédé de la revendication 10, dans lequel l'IGF mutant isolé est un mutant d'IGF-I isolé, ledit mutant d'IGF-I isolé comprenant une ou une pluralité de mutation(s) d'un résidu choisi dans le groupe constitué de lysine 27, lysine 65, lysine 68, glutamine 15 et glutamine 40, par rapport à une séquence d'acides aminés d'IGF-I de type sauvage.

12. Procédé de la revendication 9, dans lequel le membre de la famille de récepteurs d'IGF non réticulable est un mutant d' un membre de la famille de récepteurs d'IGF isolé comprenant une mutation d'un résidu lysine et/ou d'un résidu glutamine par rapport à une séquence d'acides aminés de membre de la famille de récepteurs d'IGF de type sauvage ou ;
dans lequel le membre de la famille de récepteurs d'IGF non réticulable est un mutant d'IGF1R isolé comprenant une mutation d'un résidu lysine et/ou d'un résidu glutamine.

13. Procédé de la revendication 12, dans lequel le mutant d'IGF1R isolé comprend une ou une pluralité de mutation(s) d'un résidu choisi dans le groupe constitué de lysine 159, lysine 191, lysine 498, lysine 530 et lysine 600, glutamine 14, glutamine 15, glutamine 399, glutamine 400, glutamine 287, glutamine 318, glutamine 321, glutamine 396, glutamine 511, glutamine 596, glutamine 619 et glutamine 623, par rapport à une séquence d'IGF-1R de type sauvage.
